# EUROPEAN PATENT APPLICATION

(11) **EP 1 700 919 A1**
(43) Date of publication of application: **13.09.2006**
(21) Application number: 04799520.4
(22) Date of filing: 05.11.2004
(51) Int. Cl.: C12Q 1/00, C12M 1/34, A01G 7/00, G01N 33/483, G01N 27/327

(54) **BIOSENSOR HAVING SOIL MICROORGANISM HOUSED THEREIN AND USE THEREOF**

(30) Priority: 19.11.2003 JP 2003390013
(71) Applicant: Sakata Seed Corporation, Yokohama-shi, Kanagawa 2240041 (JP); National Institute of Advanced Industrial Science and Technology, Tokyo 100-8921 (JP)
(72) Inventor: HASHIMOTO, Yoshihiro, Fujisawa-shi, Kanagawa 2520802 (JP); KARUBE, Isao, 1-1-1, Higashi, Tsukuba-shi, Ibaraki (JP)
(74) Representative: Bassil, Nicholas Charles
(86) International application number: PCT/JP2004/016422
(87) International publication number: WO 2005/049854

(57) **Abstract**

Microorganism sensors used in environment assessment technologies and the like, instead of being used to detect environmental components and determine their concentrations, were used as tools to evaluate the ability of target soil microorganisms to adapt to various field soil environments, and comparative studies were performed. As a result, the present inventors found that by using the microorganism sensors to examine the growth potential of general soil microorganisms and pathogenic microorganisms in ecosystems, the balance in the soil ecosystems could be monitored, and further, the risk of disease occurrence and the bio-controlling effect of general soil microorganisms could also be determined.

## Description

### Technical Field

The present invention relates to biosensors that use soil microorganisms, and their uses for evaluating the growth potential of soil microorganisms, assessing the risk of soil disease occurrence, and evaluating bio-controlling effects.

### Background Art

In medical fields, the development of a variety of methods for "early detection and early prevention" is required since the later the detection of diseases, in particular intractable diseases such as cancers, the more difficult their treatment becomes. The same has been said in the agricultural fields. The development of methods for "early detection and early prevention" is thought very important for soil diseases in particular.

In agricultural industries, continuous cropping is generally known to increase the chance of disease occurrence, however, continuous cropping is frequently continued in consideration of production efficiency and workability. Countermeasures are investigated if occurrence of a disease is detected; however, recovery after disease occurrence is extremely difficult. Diseases usually spread, and in severe cases, production areas are destroyed. Therefore, there is a need for earlier prediction of disease occurrence, measures to mitigate diseases, and prevention of disease spread to surrounding areas. Disease occurrence is associated with soil microorganisms, and in particular soil-borne phytopathogenic microorganisms that are direct factors of disease, and general soil microorganisms including antagonistic microorganisms. In addition, disease occurrence is also affected by environmental factors surrounding these soil microorganisms.

Soil microorganisms, especially soil-borne phytopathogenic microorganisms and general soil microorganisms (including antagonistic microorganisms), are enormous in their number and variety, and it is difficult to study them all. Current methods include counting the number of microorganisms in soil using selective media, direct or indirect microscopy using a microscope, detection using antibodies, and detection using DNA probes; however, these methods are only able to confirm the presence or absence of a microorganism of interest in a test soil at the time of sampling, or to obtain information as limited as the number of microorganisms. Moreover, the detection of soil microorganisms is problematic in that it requires laborious pretreatments, and it takes a few days to weeks to culture them and considerable time to learn the techniques, and so on.

Agricultural field sites are characterized by (1) diversity of soil types, such as sand, clay, heavy clay, volcanic ash, and alluvial soils; (2) the need to consider changes in weather conditions as well as geological factors such as latitude and topography, (3) a wide-ranging crop history, including grains such as rice and wheat, fruit vegetables such as tomato and egg plant, leafy vegetables such as broccoli, cabbage, and lettuce, root vegetables such as radish and carrot, and flowers; and (4) differences between each field site in the history of use of fertilizers, pesticides, composts, and other materials. Thus, it is very difficult to fmd a general solution for predicting disease occurrence in agricultural field sites, which are such complex systems.

Therefore, even if a single soil were selected and studied for prediction of disease occurrence, it would take several months to years and an enormous amount of time and money to perform all possible physical and chemical analyses of current soil status, studying nitrogen, phosphoric acid, potassium, other trace elements, pH, EC, three phase distribution, water permeability, water drainability, and the numbers and types of microorganisms. In such a case, the soil environment of the field site would already have changed by the time the results are obtained. Further, there remains the problem of unknown factors, which cannot be analyzed by current scientific technologies.

Recently, the field of complex system science has progressed, developing techniques for considering complex systems as total systems without disassembly, instead of analyzing components separately as in conventional approaches. For example, methods for measuring the enzyme and respiratory activities of soils have been developed; however, the conclusions that can be drawn from the results of these methods remain unclear. Methods have been proposed for estimating the diversity of soil microorganisms from pattern classifications, such as assimilation patterns, DNA patterns, and fatty acid composition patterns. Soils with a high diversity of soil microorganisms have also been reported to show disease resistance. However, this information alone is insufficient to predict disease occurrence.

Common methods for predicting disease occurrence have used the results of studies of the disease severities over the past few years to estimate whether it has been increasing or decreasing. However, there are many cases where disease occurrence is suddenly found in soils from which the disease had been absent until the previous year, or where a disease suddenly disappears from a field which had suffered from disease damage until the previous year. These cases may be explained by changes in weather conditions, the effects of newly added materials, or such; however, these are no more than speculations and several years of study are required before predictions can be made.

To meet the demands of field producers a variety of trials regarding soil disease prevention have been carried out, although the results are insufficient to predict disease occurrence. In general, fields in which a disease has developed are used to test changes in cropping types, crop rotation, cultivar improvement, pesticides, fertilizers, soil-improving materials including microorganism materials, soil sterilization, and such. However, such tests require vast test fields, and it is impractical to simultaneously perform many tests in a limited test field. In addition, considering that environmental variations make it difficult to obtain reproducible results, it is time consuming to confirm reproducibility (i.e. takes several years) and such. Further, it is extremely inefficient since data cannot be accumulated.

Field producers troubled by soil diseases have tried many microorganism materials including antagonistic microorganisms; however, the results have been very unstable and producer opinions are varied. In many cases this is because introduced antagonistic microorganisms fail to colonize the soil and plant rhizosphere due to soil conditions; or because the amount of introduced microorganism material was low compared to the very high density of pathogenic microorganisms. Yet, since the inside of a soil is a complex black box, whether or not an introduced microorganism can colonize cannot be determined without the colonization being actually carried out. In addition, the dynamics between antagonistic microorganisms and pathogenic microorganisms are virtually uninvestigated. There are a few studies where microorganism dynamics have been examined after adding a genetic marker such as drug resistance to microorganisms. However, such marked microorganisms cannot be widely used, and these studies remain at the level of investigating results, rather than predicting results.

Biosensors (Non-Patent Documents 1-3) have been mainly developed as environmental measurement technologies, as sensors for measuring biochemical oxygen demand (BOD) (Patent Documents 1-3), and detecting harmful components or nutrients in the environment, such as cyanogens (Patent Documents 4 and 5), mercury (Patent Document 6), alcohols (Patent Documents 7 and 8), detergents (Patent Document 9), phosphoric acids (Patent Documents 10 and 11), and ammonium (Patent Document 12), or measuring their concentrations. Furthermore, application of this technology is now being considered for the simple detection of food components, and the measurement of sugar levels in blood, urea concentration in urine, and such for medical diagnosis (Non-Patent Documents 4 and 5). Thus, biosensor technologies have so far been aimed at detecting components in test samples and measuring their concentrations.

Therefore, biosensors for soil microorganisms, soil diseases, or plant pathology have not been developed at all.
Patent Document 1: Japanese Patent Application Kokoku Publication No. (JP-B) S58-30537 (examined, approved Japanese patent application published for opposition)
Patent Document 2: Japanese Patent Application Kokai Publication No. (JP-A) H7-167824 (unexamined, published Japanese patent application)
Patent Document 3: JP-A H5-137597
Patent Document 4: JP-A H8-211011
Patent Document 5: JP-A H9-297105
Patent Document 6: JP-A H5-023198
Patent Document 7: JP-A H5-041999
Patent Document 8: JP-B H6-041928
Patent Document 9: JP-A H8-196295
Patent Document 10: JP-A H5-093692
Patent Document 11: JP-B H8-020401
Patent Document 12: JP-AH11-125600
Patent Document 13: JP-A H2-193059
Patent Document 14: JP-A S53-137198
Patent Document 15: JP-B H1-22898
Patent Document 16: JP-A S55-16203
Patent Document 17: JP-A S59-27255
Patent Document 18: JP-B S57-15696
Patent Document 19: JP-B S57-54742
Patent Document 20: JP-B S61-7258
Patent Document 21: JP-A S57-173745
Patent Document 22: JP-A S59-133454
Patent Document 23: JP-A H5-252994
Patent Document 24: JP-B S58-36736
Non-Patent Document 1: Biosensors & Bioelectronics, 16 (2001) 337-353
Non-Patent Document 2: Journal of Biotechnology, 15 (1990) 255-266
Non-Patent Document 3: Journal of Biotechnology, 15 (1990) 267-282
Non-Patent Document 4: Methods in Enzymology, 137 (1988) 131-138
Non-Patent Document 5: Biosensors & Bioelectronics, 16 (2001) 337-353
Non-Patent Document 6: Microbial. Ecol., 45(3) (2003) 226-236
Non-Patent Document 7: Soil. Sci. Soc. Am. J., 66(2) (2002) 498-506
Non-Patent Document 8: Analyst, 127(1) (2002) 5-7
Non-Patent Document 9: Biosensors & Bioelectronics, 16 (2001) 667-674
Non-Patent Document 10: Environ Pollut 113 (2001) 19-26
Non-Patent Document 11: Field Anal. Chem. Tech., 4(5) (2000) 239-245
Non-Patent Document 12: Soil Biol. Biochem., 32(5) (2000) 639-646
Non-Patent Document 13: Soil Biol. Biochem., 32(3) (2000) 383-388
Non-Patent Document 14: Appl. Environ. Microbiol., 69(6) (2003) 3333-3343
Non-Patent Document 15: Appl. Environ. Microbiol., 60(8) (1994) 2869-2875
Non-Patent Document 16: Can. J. Microbiol., 47 (2001) 302-308
Non-Patent Document 17: Appl. Environ. Microbiol.,67(3) (2001) 1308-1317

### Disclosure of the Invention

The present invention was achieved in view of the above circumstances. An objective of the present invention is to provide methods for quickly and simply measuring the growth potential of soil microorganisms. Furthermore, an additional objective of this invention, as an embodiment of an application of the above methods, is to provide methods of quickly and simply evaluating the risk of occurrence or spread of soil diseases caused by soil-borne phytopathogenic microorganisms. A further objective is to provide methods of evaluating the effect of general soil microorganisms in controlling soil diseases caused by soil-borne phytopathogenic microorganisms.

In order to solve the above problems, the present inventors used microorganism sensors used in environment assessment technologies and the like, and instead of using them to detect components in the environment and measure their concentrations, they used them as tools for evaluating the ability of target soil microorganisms to adapt to various field soil environments, and performed comparative studies. As a result, the present inventors found that by using the microorganism sensors to examine the growth potential of general soil microorganisms and pathogenic microorganisms in ecosystems, the balance in soil ecosystems could be monitored, and further, the inventors found that the risk of disease occurrence and the bio-controlling effect of general soil microorganisms could also be determined.

In particular, when determining the above, the electrode response ratios of general soil microorganisms/pathogenic microorganisms, which were measured using sensor units storing soil-borne phytopathogenic microorganisms and those storing general soil microorganisms, served as an effective index.

Specifically, when the electrode response ratio is high, the growth of general soil microorganisms exceeds that of pathogenic microorganisms, and the balance in the soil ecosystem is therefore predicted to shift toward a state where there are more general soil microorganisms than pathogenic microorganisms. Further, the risk of disease occurrence or spread is predicted to be low, and the biocontrol effect of general soil microorganisms in the soil is predicted to be high.

On the other hand, when the electrode response ratio is low, the growth rate of pathogenic microorganisms is predicted to exceed that of general soil microorganisms, and the balance in the soil ecosystem is predicted to shift toward a state where there are more pathogenic microorganisms than general soil microorganisms. Therefore, the risk of disease occurrence or spread is predicted to be high and the biocontrol effect of general soil microorganisms in the soil is predicted to be low.

Further, the risk of disease occurrence can be more precisely predicted by analyzing the current conditions and considering the number of pathogenic microorganisms and general soil microorganisms, as well as the state of disease-occurrence of the agricultural field.

In particular, this technology is extremely useful because it enables early soil diagnosis regarding the possibility of disease occurrence in agricultural fields where there has been no disease occurrence.

Thus, more specifically, the present invention provides the following inventions:
[1] a method for measuring the growth potential of a soil microorganism in a soil, comprising the steps of:
   (a) contacting a soil suspension of a soil to be measured with multiple sensors, each of which comprise a ucomprising an oxygen electrode, a housing section that stores a soil microorganism, and an immobilizing member, wherein the housing section of each sensor stores a different soil microorganism; and
   (b) measuring the differences in a decrease or rate of decrease of output electric current for each of the sensors;
[2] a method for evaluating the risk of the occurrence or spread of a soil disease caused by a soil-borne phytopathogenic microorganism, comprising the steps of:
   (a) contacting a soil suspension of a soil to be measured with a sensor which comprises a ucomprising an oxygen electrode, a housing section that stores a general soil microorganism, and an immobilizing member, and with a sensor which comprises a ucomprising an oxygen electrode, a housing section that stores a soil-borne phytopathogenic microorganism, and an immobilizing member; and
   (b) measuring a decrease or rate of decrease in output electric current for each of the sensors,
   wherein the risk of occurrence or spread of the soil disease caused by the soil-borne phytopathogenic microorganism in the soil is determined to be low when the decrease or rate of decrease of output current in the case of the general soil microorganism is significantly higher than that in the case of the soil-borne phytopathogenic microorganism;
[3] a method for evaluating an effect of a general soil microorganism in controlling a soil disease caused by a soil-borne phytopathogenic microorganism, comprising the steps of:
   (a) contacting a soil suspension of a soil to be measured with a sensor which comprises a ucomprising an oxygen electrode, a housing section that stores a general soil microorganism, and an immobilizing member, and also contacting the soil suspension of the soil to be measured with a sensor which comprises a ucomprising an oxygen electrode, a housing section that stores a soil-borne phytopathogenic microorganism, and an immobilizing member; and
   (b) measuring a decrease or rate of decrease in output electric current for each of the sensors,
   wherein the general soil microorganism is determined to have an effect in controlling the soil disease caused by the soil-borne phytopathogenic microorganism in the soil when the decrease or rate of decrease of output current in the case of the general soil microorganism is significantly higher than that in the case of the soil-borne phytopathogenic microorganism;
[4] a kit used for the method of [1], comprising multiple sensors each of which comprise a unit comprising an oxygen electrode, a housing section that stores a soil microorganism, and an immobilizing member, wherein the housing section of each sensor stores a different soil microorganism; and
[5] a kit used for the method of [2] or [3], comprising a sensor that comprises a ucomprising an oxygen electrode, a housing section that stores a general soil microorganism, and an immobilizing member; and comprising a sensor which comprises a ucomprising an oxygen electrode, a housing section that stores a soil-borne phytopathogenic microorganism, and an immobilizing member.

### Brief Description of the Drawings

Fig. 1 shows the response of a microorganism sensor storing *Fusarium* fungi to addition of a substrate (PD broth). The vertical axis shows the electric current (nA) of the microorganism sensor, and the horizontal axis shows the volume (µl) of PD broth added.
Fig. 2 shows the response of a microorganism sensor storing *Plasmodiophora brassicae* to yeast extract. The vertical axis shows the electric current (nA) of the microorganism sensor, and the horizontal axis shows the volume (ml) of immobilized fungus.

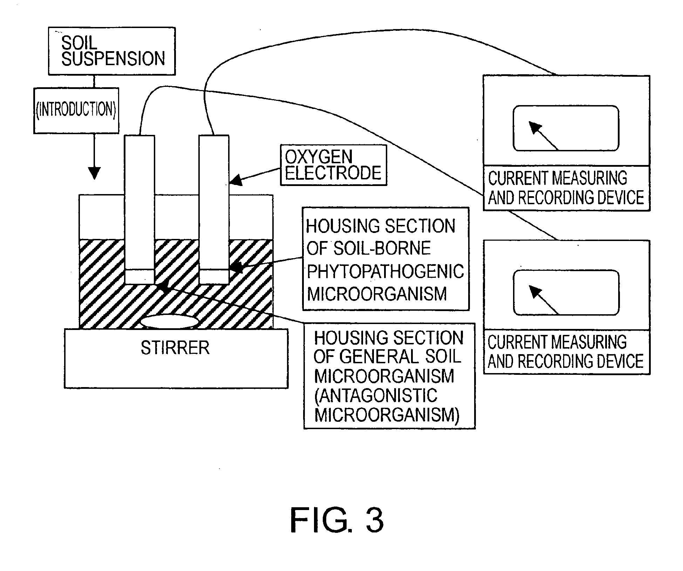

Fig. 3 shows the structure of a microorganism sensor for predicting the occurrence of a soil disease.

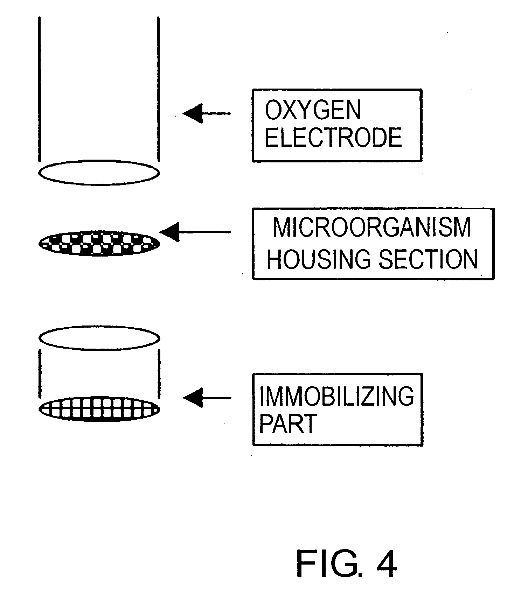

Fig. 4 shows the structure of a microorganism sensor unit.

### Best Mode for Carrying Out the Invention

The present invention provides methods for measuring the growth potential of soil microorganisms in soils. The methods comprise the steps of (a) contacting a suspension of a test soil with multiple sensors that each comprise a ucomprising an oxygen electrode, a housing section that stores a soil microorganism, and an immobilizing member, wherein the housing section of each sensor comprises a different soil microorganism; and (b) measuring the differences in the decrease or rate of decrease of output electric current for each of the sensors.

The types of soil microorganisms used in this invention are not particularly limited, and may be any microorganism that can be isolated and cultured by conventional techniques. Moreover, unculturable microorganisms such as *Plasmodiophora brassicae* may also be used after being grown and isolated using a growth method that utilizes plant bodies.

The types of soil microorganisms preferably include those described below:
First, the soil-borne phytopathogenic microorganisms of the present invention are classified into bacteria, actinomycetes, and fungi.
Soil-borne phytopathogenic bacteria include *Ralstonia solanacearum,* soft rot bacteria (*Erwinia, Pseudomonas),* crown gall bacteria *(Agrobacterium),* etc.
Soil-borne phytopathogenic actinomycetes include common scab bacteria *(Streptomyces),* etc.
Soil-borne phytopathogenic fungi include damping-off fungi *(Pythium, Rhizoctonia,* etc.), *Plasmodiophora brassicae,* late blight fungi (*Phytophthora, Verticillium, Fusarium,* and *Rhizoctonia*), root rot fungi *(Helicobasidium,* and *Rosellinia),* southern blight fungi *(Corticium),* brown root rot fungi (*Pyrenochaeta*), stripe fungi (*Cephalosporium*), dry rot fungi *(Cylindrocarpon),* etc.
In addition, general soil microorganisms of the present invention are classified into antagonistic bacteria and antagonistic fungi, (referred to above as antagonistic microorganisms), general soil bacteria, general soil actinomycetes, general soil fungi, and so on (Soil Microorganisms (1981), Japanese Society of Soil Microbiology (ed.), Hakuyusha).
Antagonistic bacteria include *Bacillus,* non-pathogenic *Agrobacterium, Enterobacter, Pseudomonas, Xanthomonas, Streptomyces,* non-pathogenic *Erwinia, Pasteuria,* etc.
Antagonistic fungi include *Aspergillus,* non-pathogenic *Fusarium, Gliocladium, Penicillium, Pythium, Trichoderma, Phoma, Talaromyces,* etc.
General soil bacteria include *Acetobacter, Alcaligenes, Bacillus, Burkholderia, Corynebacterium, Flavobacterium, Gluconobacter, Lactobacillus, Mycobacterium, Micrococcus, Proteus, Pseudomonas, Rhizobium, Rhodococcus, Sphingomonas, Streptococcus, Zymomonas,* etc.
General soil actinomycetes include *Streptomyces, Actinomadura, Glycomyces, Nocardia, Saccharomonospora, Streptoverticillium,* etc.
General soil fungi include *Aphanomyces, Aspergillus, Candida, Cladosporium, Mucor, Penicillium, Phytophthora, Rhizopus, Trichoderma, Torula,* etc.

The culture conditions for the general microorganisms of the present invention may be those described in experimental books (Shinpen Dojoubiseibutsujikkenhou (New Edition Soil Microorganisms Experimental Methods) (1997), Japanese Society of Soil Microbiology (ed.), Yokendo) or such. For example, culture media may be meat extract medium, LB medium, potato dextrose medium (PD medium) and such, and cultures may be performed in containers such as dishes, test tubes, flasks, and jar fermenters under conditions such as standing, shaking, and stirring. No special culture conditions are necessary. In addition, unculturable microorganisms can also be used if they can be grown in a particular part of a plant, such as root knot, and isolated on a large scale.

The oxygen electrodes for the biosensors used in the present invention can be those in general use, and commercially available galvanic and polarographic types may be used.

The microorganism housing sections constituting the biosensors preferably minimize leakage of microorganisms from the housing section. The housing sections are made of membranes with a mesh size permeable to water, volatile substances such as oxygen dissolved in water, organic substances influencing microorganism respiratory activity, substances that inhibit respiratory activity (water-dissolved substances and particles smaller than the microorganisms), and such. The housing sections may be made of a material with enough strength to retain the microorganisms. For example, nitrocellulose membranes or acetyl cellulose membranes with a pore size of 0.45 µm may be used.

The methods for storing the microorganisms include methods in which microorganism suspensions are dropped on membranes of nitrocellulose, acetylcellulose, nylon, or such, and then water and water-soluble substances are removed by vacuum from the bottom or pressure from the top to immobilize the microorganisms onto filters by adsorption; and methods in which microorganisms are immobilized in gels of calcium alginate or such, and then thin film sections are prepared.

The immobilizing parts constituting the biosensors are not particularly limited as long as they can hold a microorganism housing section as close as possible to an electrode, and so long as they do not prevent the diffusion of volatile substances such as oxygen, and organic substances and inhibitors that influence microorganism respiratory activity, from the soil suspension to the immobilized microorganisms and electrode. For example, when the part under the membrane on which the microorganisms are immobilized is made of nylon net or steel mesh, water soluble substances and minute particulate matter can permeate. Further, for example, the sides of the immobilizing part are made of plastic or such, and are threaded or alternatively fixed to the electrode using O-rings or tubes, etc.

The soil suspensions to be contacted with the biosensors are preferably supernatants obtained by collecting appropriate amounts of soils (for example, 10-1,000 g) from agricultural field sites, adding appropriate volumes of water, solvent, or buffer(for example, 1-100 times the amount of soil sample), mixing by stirring well, and then performing centrifugation or filtration.

To detect the output currents, the microorganism sensors that store the microorganisms are contacted with an appropriate volume (for example, 0.01-1,000 ml) of a soil suspension, and the decrease or rate of decrease of the output current is measured whilst stirring. As a result, the larger the decrease or rate of decrease in output current, the greater the respiratory activity of the soil microorganism and the more oxygen is being consumed: i.e. the higher the growth potential of the microorganisms in the soil (the soil environment is suitable for their growth). A small decrease or rate of decrease in output current means low respiratory activity of the microorganisms and low oxygen consumption, indicating that the growth potential of the microorganisms in the soil is low (i.e. the soil environment is not preferable for their growth). That is, the soil has a low content of organic substances to feed the microorganisms or it contains substances that inhibit their respiratory activity, etc.

By performing similar studies using microorganism sensors in which each of multiple microorganisms are separately stored, users can determine what kinds of microorganisms have relatively high growth potential (are capable of preferential growth) in the soil of a field site. In addition, when introducing antagonistic microorganisms to a soil, users can determine whether the antagonistic microorganism will be able to adapt to the soil environment. Moreover, it is also possible to investigate the dynamics of multiple microorganisms in compost fermentation processes.

In the above-described methods for measuring the growth potential of soil microorganisms, the use of general soil microorganisms and a soil-borne phytopathogenic microorganism as different microorganisms enables evaluation of the risk of occurrence or spread of a soil disease caused by the phytopathogenic microorganism.

Thus, the present invention further provides methods of evaluating the risk of occurrence or spread of soil diseases caused by soil-borne phytopathogenic microorganisms, comprising the steps of (a) contacting test soil suspensions with sensors that comprise a unit comprising an oxygen electrode, a housing section that stores a general soil microorganism, and an immobilizing member, and then contacting the test soil suspensions to sensors that comprise a ucomprising an oxygen electrode, a housing section that stores a soil-borne phytopathogenic microorganism, and an immobilizing member, and (b) measuring the decreases or rates of decrease in the output electric currents of each sensor.

In these methods, when the decrease or rate of decrease in output current for a general soil microorganism is significantly higher than that for a soil-borne phytopathogenic microorganism, the risk of occurrence or spread of a soil disease caused by the phytopathogenic microorganism in the measured soil is determined to be low. On the other hand, when the decrease or rate of decrease in output current for the general soil microorganism is significantly lower than that for the soil-borne phytopathogenic microorganism, the risk of occurrence or spread of a soil disease caused by the phytopathogenic microorganism in the measured soil is determined to be high.

Similarly, it is also possible to evaluate the effect of general soil microorganisms in controlling soil diseases caused by soil-borne phytopathogenic microorganisms by (a) contacting a suspension of a particular soil to a sensor that comprises a ucomprising an oxygen electrode, a housing section that stores a general soil microorganism, and an immobilizing member, and to a sensor that comprises a ucomprising an oxygen electrode, a housing section that stores a soil-borne phytopathogenic microorganism, and an immobilizing member, and (b) measuring the decreases or rates of decrease in the output electric current of each sensor. Thus, the present invention also provides methods for evaluating the effects of general soil microorganisms in controlling soil diseases caused by soil-borne phytopathogenic microorganisms.

In these methods, when the decrease or rate of decrease in output current for a general soil microorganism is significantly higher than that for a soil-borne phytopathogenic microorganism, the general soil microorganism is determined to have an effect in controlling soil diseases caused by the soil-borne phytopathogenic microorganism in the measured soil. On the other hand, when the decrease or rate of decrease in output current for the general soil microorganism is significantly lower than that for the soil-borne phytopathogenic microorganism, the general soil microorganism is determined to have no effect in controlling soil diseases caused by the phytopathogenic soil microorganism in the soil.

The decreases or rates of decrease in output current for general soil microorganisms may be compared with those for soil-borne phytopathogenic microorganisms by evaluation using an electrode response ratio (general soil microorganism/pathogenic microorganism). For example, when the electrode response of a microorganism sensor storing a general soil microorganism (including antagonistic microorganisms) is significantly higher than that of a sensor storing a pathogenic microorganism, it is predicted that the general soil microorganism (including antagonistic microorganisms) will grow faster than the pathogenic microorganism, and that the risk of future disease occurrence is in decline. In this case, the general soil microorganism may be determined to have a controlling effect in the soil.

Herein, "the electrode response of a microorganism sensor storing a general soil microorganism is significantly higher than that of a microorganism sensor storing a pathogenic microorganism" means normally an electrode response ratio of 0.4 or higher, preferably 0.6 or higher, and more preferably 2.0 or higher. The reliability of this value can be improved by correction based on the current disease severities and the results of measuring the density or abundance ratio of pathogenic microorganisms and general soil microorganisms, as described below.

When there is no difference in the electrode response between a microorganism sensor storing a general soil microorganism (including antagonistic microorganisms) and a sensor storing a pathogenic microorganism, the pathogenic microorganism and the general soil microorganism (including antagonistic microorganisms) are predicted to exhibit almost the same growth, and the risk of future disease occurrence is predicted to remain the same as the current state. In this case, the controlling effect of the general soil microorganism may be determined to be unclear.

Alternatively, when the electrode response of a microorganism sensor storing a general soil microorganism (including antagonistic microorganisms) is significantly less than that of a sensor storing a pathogenic microorganism, it indicates that the pathogenic microorganism is growing faster and growth of the general soil microorganism is slow. Thus, suppression of the pathogenic microorganism is predicted to be difficult, and the risk of future disease occurrence can be predicted to further increase. In this case, the general soil microorganism can be determined to have a small controlling effect in the soil.

The precision with which the risk of disease occurrence can be predicted may be improved by using the biosensors to evaluate the electrode response ratio results after assessing the current situation based on the current disease severities and the results of measuring the density or abundance ratio of pathogenic microorganisms and general soil microorganisms. For example, when the density or abundance ratio of pathogenic microorganisms is significantly greater than that of general soil microorganisms, the controlling effect may become relatively smaller (see Table 29). In addition, when the disease severity is high, the protective values tend to be low (see the disease severities and protective values in the two tests (1) and (2) in each of Tables 24, 25, and 27). For example, under conditions of severe occurrence, where the disease severity is 90% or higher, it is difficult to exhibit a disease-reducing effect in a short time, even if the electrode response ratio is significantly high (Example 12).

The disease severity of a field soil may be evaluated by cultivating crops, then examining disease occurrence status, as shown in Examples 11 and 12. Further, the number of pathogenic microorganisms and general soil microorganisms (including antagonistic microorganisms) in the soil can be assessed by determining the abundance ratio of each microorganism of interest in the soil using standard dilution plate methods with selective media, or by direct microscopy, antibody methods, methods using DNA probes, or such. In addition, pathogenic microorganisms or dominant species may be selected and isolated from colonies obtained by dilution plate methods, and then immobilized in microorganism sensors of the present invention. In addition, the number of antagonistic microorganisms to be introduced to an agricultural field may be easily determined using standard dilution plate methods or such, although some materials record the density of the microorganisms.

Further, the present invention may be applied as described below to prepare suggestions (formulae) for soil improvement regarding strategies for disease reduction.

The chemical, physical, and biological characteristics of field soils in pots can be improved by adding fertilizers, pesticides, soil-improving materials and such; adjusting soil pH, the content of organic substances, and such; and improving water permeability, water retentivity, air permeability, biological characteristics, and such. These improvements, however, are simultaneously accompanied by changes in the growth speed of pathogenic microorganisms and general soil microorganisms (including antagonistic microorganisms). A microorganism sensor that stores a pathogenic microorganism can be used to easily determine what or how much material to add to reduce the growth speed of a pathogenic microorganism. Similarly, a microorganism sensor that stores a general soil microorganism (including antagonistic microorganisms) can be used to easily determine whether or not the growth speed of the general soil microorganism (including antagonistic microorganisms) will increase.

Based on the above results, it is possible to clearly optimize the conditions under which the electrode response ratio (general soil microorganism/pathogenic microorganism) of both microorganism sensors is largest; that is, soil improvement can be optimized (target values) such that the rate of oxygen consumption is slow for a pathogenic microorganism, and fast for an antagonistic microorganism. Suggestions (formulae) tailored to improvement of a field's soil environment can thus be made.

Furthermore, the present invention provides kits used for the above-described methods of the present invention. For example, kits used for the methods for measuring the growth potential of soil microorganisms comprise multiple sensors, each of which comprises a unit comprising an oxygen electrode, a housing section that stores a soil microorganism, and an immobilizing member, where the housing section of each sensor stores a different soil microorganism.

Furthermore, in the case of kits used for the methods of evaluating the occurrence or spread of soil diseases caused by soil-borne phytopathogenic microorganisms, or used for the methods for evaluating the effects of general soil microorganisms in controlling soil diseases caused by soil-borne phytopathogenic microorganisms, the kits comprise a sensor which comprises a ucomprising an oxygen electrode, a housing section that stores a general soil microorganism, and an immobilizing member, and also comprise a sensor which comprises a ucomprising an oxygen electrode, a housing section that stores a soil-borne phytopathogenic microorganism, and an immobilizing member.

These kits may further comprise an instruction manual or the like.

All the prior art references cited herein are incorporated herein by reference.

### Examples

Herein below, the present invention will be specifically described with reference to Examples, but it is not to be construed as being limited thereto.

### [Example 1] Preparation of a microorganism sensor storing Fusarium fungi and its response.

The SN3B strain of *Fusarium oxysporum* f. sp. *Lactucum* was used as a representative phytopathogenic microorganism.

The SN3B strain, which was isolated in Seba district, Nagano prefecture, Japan, and subsequently introduced with a gene deletion marker, nit⁻, at the Nagano Vegetable and Ornamental Crops Experiment Station, was provided by the Nagano Chushin Agricultural Experiment Station.

The *Fusarium* fungi were cultured at 25°C for one week in a potato dextrose (PD) broth medium. After removing the hyphae by filtration through gauze, spores were collected by centrifugation, and twice washed with 20 mM phosphate buffer (pH 7.0). The density of the fungi at this point was OD₆₆₀ = 1.33. The spores were counted using a hemocytometer and the spore count was determined to be 1.0 x 10⁶/ml.

Fungi in a 2 ml culture solution were immobilized on a 0.45 µm nitrocellulose filter by aspiration, then placed in a microorganism sensor unit (2.0 x 10⁶ spores/filter were placed).

Fifty ml of 20 mM phosphate buffer (pH 7.0) was placed in a beaker and brought to 30°C in a water bath. The microorganism sensor storing the *Fusarium* fungi was inserted, and the buffer was slowly stirred with a stirrer. (Note: since the rate of oxygen supply from the air changes depending on stirring speed, the experiments were performed with a fixed speed of stirrer rotation (rotating speed: about 100-200 rpm)).

The electric current (nA) generated at the membrane of the microorganism electrode was measured.

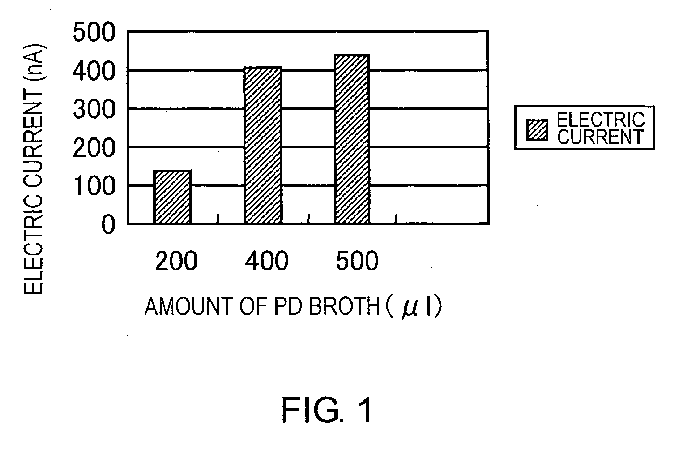

The baseline was first stabilized, and then various organic substances were added and the response of the microorganism sensor storing the *Fusarium* fungi was examined. The results are shown in Table 1 and Fig. 1.

The results show that the microorganism sensor responded to media such as PD broth, which is actually used for culture, much better than to the addition of a single saccharide, such as glucose or fructose. The addition of 500 µl of PD broth per 50 ml exhibited the greatest response.

**[Table 1] Response of a microorganism sensor storing Fusarium fungi to added substrates**

| Substrate | Volume added (µl) | Electric current decrease (Δ n A) | Relative value (%) |
|---|---|---|---|
| 10% glucose | 200 | 40 | 30 |
| 10% fructose | 200 | 35 | 26 |
| L broth | 200 | 125 | 93 |
| PD broth* | 200 | 135 | 100 |
| PD broth | 400 | 408 | 302 |
| PD broth | 500 | 435 | 322 |

| | | | |
|---|---|---|---|
| *The relative value was calculated by setting as 100 the decrease in current when 200 µl of PD broth was added. | | | |

Next, 20 mM phosphate buffer (pH 7.0) was added to various soil and compost samples, mixed well, and incubated at room temperature. The samples were then centrifuged to collect supernatant solution. The results are shown in Table 2. A microorganism sensor storing *Fusarium* fungi was inserted in 50 ml of 20 mM phosphate buffer (pH 7.0) kept at 30°C. After the baseline was confirmed to be stable, 5 ml of the supernatant solutions from various soil and compost samples was added, and the responses of the microorganism sensor storing *Fusarium* fungi was examined.

The results shown in Table 2 indicate that the microorganism sensor storing *Fusarium* fungi responded well to the various soil and compost samples. In black soil, there were a few organic substances available to the *Fusarium* fungi when the incubation time was short (around two hours), whereas no electrode response was observed after two days of incubation. This could be attributed to two things: a decrease in the organic substances able to be readily consumed by the *Fusarium* fungi; or a bacteriostatic action (unknown factor) of the soil that inhibits the fungi's growth or respiration. However, visual observations showed the supernatant was clearly more dense after two days of incubation, was and was thus considered to have a higher organic content. Thus, the above result suggests a strong possibility that bacteriostatic substances (unknown factors) derived from the black soil emerged during the two-day incubation. In addition, the electrode response was very small in Akadama soil, which clearly has a low organic content. Early stage oxygen consumption by the *Fusarium* fungi clearly tended to be greater in the organic-rich samples, such as completely matured compost (chicken droppings), herbal compost, and leaf mold. The results also confirmed that the microorganism sensor storing the *Fusarium* fungi showed a sufficient response to ordinary soil samples such as those taken from the Chougo farm.

Further, it is better to extract from soil and compost samples by vigorously shaking the samples for two hours instead of ineffectively leaving them to stand for two days without shaking.

**[Table 2] Response of a microorganism sensor storing Fusarium fungi to various soil and compost samples**

| Sample | (Product name) | Dilution | Incubation time | Electric current decrease (Δ n A) | Relative value (%) |
|---|---|---|---|---|---|
| Black soil | | 1 : 5 | 2 hr shake | 49 | 24 |
| Black soil | | 1 : 5 | 2 day standing | 0 | 0 |
| Completely matured compost (chicken droppings) | Baitech-Bioace | 1 : 5 | 2 hr shake | 338 | 168 |
| Completely matured compost (chicken droppings)* | Baitech-Bioace | 1 : 5 | 2 day standing | 201 | 100 |
| Chougo farm open-field soil | | 1 : 5 | 2 day standing | 30 | 15 |
| Chougo farm greenhouse soil | | 1 : 5 | 2 day standing | 86 | 43 |
| Herbal compost | Tsumuland | 1 : 5 | 2 day standing | 279 | 139 |
| Leaf mold | Bioace-soft | 1 : 5 | 2 day standing | 118 | 59 |
| Akadama soil | | 1 : 5 | 2 day standing | 21 | 10 |

| | | | | | |
|---|---|---|---|---|---|
| * The relative value was calculated by setting at 100 the decrease in current for completely matured compost (chicken droppings), product name: Baitech-Bioace, two days standing. | | | | | |

### [Example 2] Preparation of a microorganism sensor storing a Bacillus bacterium and its response.

The K12N strain of *Bacillus cereus* was used as representative antagonistic microorganism.

The *Bacillus* bacteria were inoculated in L broth media, then incubated for 24 hours at 30°C. The bacteria were collected by centrifugation and washed twice with 20 mM phosphate buffer (pH 7.0). The microorganism density was OD₆₆₀ = 0.19. The viable cell count was calculated to be about 6.0 x 10⁶ cfu/ml.

Two ml of culture was immobilized on a 0.45 µm nitrocellulose filter by aspiration, and then placed in the microorganism sensor unit (1.2 x 10⁷ cfu of bacteria was stored per filter).

Fifty ml of 20 mM phosphate buffer (pH 7.0) was placed in a beaker, and brought to 30°C in a water bath. The microorganism sensor storing the *Bacillus* bacteria was inserted, and the solution was slowly stirred with a stirrer.

The electric current (nA) generated at the membrane of the microorganism electrode was measured.

The base line was first stabilized, and then various organic substances were added and the response of the microorganism sensor storing the *Bacillus* bacteria was confirmed (Table 3).

The results showed that the microorganism sensor responded to all substances except albumin. It is though that the sensor did not respond to albumin because the albumin did not dissolve in water well. In addition, the response of the microorganism sensor to substrates such as yeast extract, tryptone and peptone, which are rich in amino acids and proteins actually used for *Bacillus* bacteria culture, was better than the response to glucose.

**[Table 3] Response of a microorganism sensor storing a Bacillus bacterium to added substrates.**

| Substrate | Volume added (µl) | Electric current decrease (Δ n A) | Relative current (%) |
|---|---|---|---|
| 10% glucose | 50 | 38 | 9 |
| 10% glucose | 100 | 138 | 34 |
| 10% glucose | 150 | 125 | 31 |
| 10% glucose | 200 | 150 | 38 |
| 10% glucose | 300 | 150 | 38 |
| 10% glucose | 400 | 200 | 50 |
| 1 % yeast extract | 250 | 625 | 156 |
| 1 % tryptone | 250 | 450 | 113 |
| 1% albumin | 250 | 0 | 0 |
| 1 % peptone | 250 | 638 | 159 |
| L broth* | 250 | 400 | 100 |

| | | | |
|---|---|---|---|
| *The relative value was calculated by setting as 100 the decrease in current when L broth was added. | | | |

Next, 20 mM phosphate buffer (pH 7.0) was added to various soil and compost samples, mixed well, and incubated at room temperature. The samples were then centrifuged to collect supernatant solutions. The microorganism sensor storing the *Bacillus* bacterium was inserted in 50 ml of 20 mM phosphate buffer (pH 7.0) kept at 30°C. After confirming that the baseline was stable, 5 ml of the supernatant solutions from various soil and compost samples was added to the buffer, and the response of the microorganism sensor storing the *Bacillus* bacterium was examined. The results are shown in Table 4.

The results show that the microorganism sensor storing the *Bacillus* bacterium responded to completely matured compost (chicken droppings) (Product name: Baitech-Bioace, Sakata Seed Co.), leaf mold (Product name: Bioace-soft, Sakata Seed Co.), herbal compost (Product name: Tsumuland, Tsumura & Co.), and black soil (a commercial product), which are rich in organic substances. In addition, the microorganism sensor also responded to the field soil samples, i.e. soil samples from open fields and greenhouses on the Chougo farm. In contrast, the biosensor did not respond to Akadama soil (a commercial product), which contains less organic substances.

**[Table 4] Response of a microorganism sensor storing a Bacillus bacterium to various soil and compost samples**

| Sample | (Product name) | Dilution | Incubation time | Electric current decrease (Δ n A) | Relative value (%) |
|---|---|---|---|---|---|
| Black soil | | 1 : 5 | 2 hrs | 45 | 45 |
| Completely matured compost (chicken droppings)* | Baitech-Bioace | 1 : 5 | 2 hrs | 100 | 100 |
| Chougo farm open-field soil | | 1 : 5 | 2 hrs | Trace | Trace |
| Chougo farm greenhouse soil | | 1 : 5 | 2 hrs | 63 | 63 |
| Herbal compost | Tsumuland | 1 : 5 | 2 hrs | 63 | 63 |
| Leaf mold | Bioace-soft | 1 : 5 | 2 hrs | 75 | 75 |
| Akadama soil | | 1 : 5 | 2 hrs | 0 | 0 |

| | | | | | |
|---|---|---|---|---|---|
| * The relative value was calculated by setting as 100 the decrease in current for completely matured compost (chicken droppings), product name: Baitech-Bioace. | | | | | |

### [Example 3] Comparison of the response of the microorganism sensor storing the Bacillus bacterium and that storing the Fusarium fungi

*Bacillus* bacterial cells and a spore suspension of *Fusarium* fungi, cultured to the logarithmic growth phase as in Examples 1 and 2, were collected, washed, and then their absorbance at 660 nm was measured. The density of *Bacillus* bacteria was 0.43, and that of *Fusarium* fungi was 0.1. 1.0 ml of each microorganism suspension was immobilized on a nitrocellulose filter by aspiration, and then placed in the microorganism sensor units.

The number of viable microorganisms was separately measured using the dilution plate method. The number of *Bacillus* bacteria and *Fusarium* fungi in each sensor unit was 1.4 x 10⁷ cfu/filter and 1.0 x 10⁵ cfu/filter, respectively.

The density of general *Bacillus* bacteria and *Fusarium* fungi in ordinary soil is 10⁵-10⁶ cfu and 10²-10³ cfu per gram of soil, respectively. Thus, the amount of *Bacillus* bacteria in the sensor corresponded to that in about 14-140 g of soil. The amount of *Fusarium* fungi in the sensor corresponded to that in about 100-1000 g of soil.

The results of examining the responses to various substrates is shown in Table 5. As the results in Table 5 clearly indicate, the sensor storing the *Bacillus* bacterium showed a better electrode response to yeast extract, L broth, and completely matured compost "Baitech-Bioace" than the sensor storing *Fusarium* fungi.

**[Table 5] Comparison of the response of a microorganism sensor storing a Bacillus bacterium with that storing Fusarium fungi**

| Substrate | Concentration | Volume added | *Bacillus* | *Fusarium* |
|---|---|---|---|---|
| Yeast extract | 1% aqueous solution | 100µl | 625 (ΔnA) | 147(ΔnA) |
| L broth | | 100µl | 400 | 123 |
| Completely matured compost (chicken droppings) "Baitech-Bioace" | 1/5 suspension | 5.0ml | 184 | 118 |

### [Example 4] Effect of the extraction time of soil and compost on the response of a microorganism sensor

The spore suspension of *Fusarium* fungi in Example 1 was immobilized on a nitrocellulose filter by aspiration, and placed in a microorganism sensor unit (storing 0.35 ml of spore suspension with OD₆₆₀ = 1.45; about 5 x 10⁵ cfu/filter).

"Baitech-Bioace", a commercially-available completely matured compost of chicken droppings, was used as the compost sample. A commercially available "black soil" was used as the soil sample. 40 ml of 10 mM phosphate buffer (pH 7.0) was added to 10 g of each sample, and this was shaken for three hours or one, two, or three days. The samples were centrifuged at 3,000 rpm for 30 minutes to collect the supernatant suspensions. After confirming there was nc problem with the response of the microorganism sensors when using yeast extract as a substrate, their responses were examined using the compost and soil sample extracts.

The results are shown in Table 6.

**[Table 6] Effect of extraction time on microorganism sensor response to compost and soil samples**

| Sample | Extraction time | Δ n A |
|---|---|---|
| Completely matured compost of chicken droppings (Baitech-Bioace) | 3 hrs | 235 |
| | 1 day | 225 |
| | 2 days | 224 |
| | 3 days | 376 |
| Black soil | 3 hrs | 48 |
| | 1 day | 77 |
| | 2 days | 81 |

The results in Table 6 show that stable results with less data fluctuation can be achieved when extracting compost and soil by shaking the samples for one to two days.

### [Example 5] Preparation of microorganism sensors that store a variety of biological pesticides, microorganism materials, and soil microorganisms, and their responses.

**[Table 7] Summary of used materials and sources**

| Microorganism (genus) | Product name (Strain No.) | Class | Supplier/Manufacturer |
|---|---|---|---|
| *Trichoderma* | Hardin-L | Microorganism material | Kawata Kogyo |
| *Pseudomonas* | Cerafarm | Microorganism material | Central Glass Co. Ltd |
| *Bacillus* | Botokiller | Biological pesticide | Idemitsu Kosan Co. Ltd. |
| *Pseudomonas* | HAI00377 | | Hyogo Prefecture Agricultural Research Center |
| *Streptomyces* | Mycostop | Microorganism material | Kemira Agro Oy |
| *Pythium* | MMR2 | | Hokkaido Agricultural Experiment Station |

Each microorganism was activated by overnight culturing at 25°C in L broth with shaking. The density of microorganisms was calculated by using a spectrometer to measure absorbance at 660 nm. Then, the microorganisms were collected, washed, immobilized on a filter by aspiration, and placed in sensor units.

The response of each microorganism sensor storing a different microorganism was examined using various substrates. The results are shown in Tables 8-10.

As shown in Tables 8 and 9, all the microorganisms responded when L broth or yeast extract was used as a substrate, although the strength of response varied depending on the kind of microorganisms stored in the microorganism sensor.

As shown in Tables 10 and 11, all the microorganism sensors responded to compost and soil samples such as completely matured compost (chicken droppings) and black soil.

**[Table 8] Response of microorganism sensors storing various microorganisms to L broth**

| Microorganisms (genus) | Product name or (Strain No.) | OD660 | Δ n A |
|---|---|---|---|
| *Trichoderma* | Hardin-L | 0.6 | 107 |
| *Pseudomonas* | Cerafarm | 0.43 | 377 |
| *Bacillus* | Botokiller | 1.94 | 37 |
| *Pseudomonas* | HAI00377 | 0.2 | 341 |
| *Streptomyces* | Mycostop | 0.1 > | 120 |
| *Pythium* | MMR2 | 0.1 > | 61 |

**[Table 9] Response of microorganism sensors storing various microorganisms to yeast extract**

| Microorganisms (genus) | Product name (No.) | OD660 | Δ n A |
|---|---|---|---|
| *Trichoderma* | Hardin-L | 0.6 | 106 |
| *Bacillus* | Botokiller | 1.94 | 52 |

**[Table 10] Response of microorganism sensors storing various microorganisms to completely matured compost of chicken droppings (Product name: Baitech-Bioace)**

| Microorganisms (genus) | Product name (No.) | OD660 | Δ n A |
|---|---|---|---|
| *Trichoderma* | Hardin-L | 0.6 | 21 |
| *Pseudomonas* | Cerafarm | 0.43 | 182 |
| *Pseudomonas* | HAI00377 | 0.2 | 104 |
| *Pythium* | MMR2 | 0.1 > | 10 |

**[Table 11] Response of microorganism sensors storing various microorganisms to black soil (commercial soil)**

| Microorganisms (genus) | Product name (No.) | OD660 | Δ n A |
|---|---|---|---|
| *Streptomyces* | Mycostop | 0.1 > | 29 |
| *Pseudomonas* | Cerafarm | 0.43 | 62 |
| *Pythium* | MMR2 | 0.1 > | 13 |

### [Example 6] Preparation of a microorganism sensor storing a Brassicaceae clubroot fungus and its response.

Since a *Brassicaceae* clubroot fungus *(Plasmodiophora brassicae)* is an unculturable microorganism, root knots developed in the roots of Chinese cabbages were collected from an agricultural field where root-knot disease had developed. Spore suspensions were prepared by squashing the root knots using a mixer, then collecting and washing the microorganisms. Spore density was measured using a hemocytometer and found to be 2.8 x 10⁹ spores/ml.

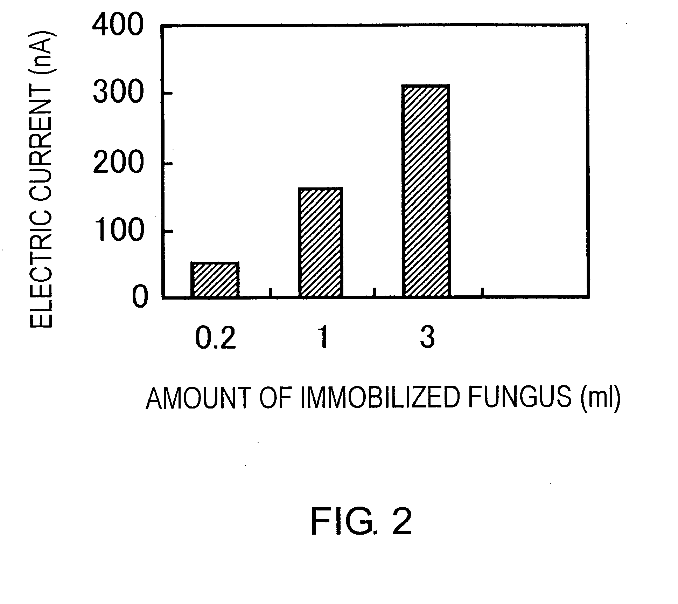

The above spore suspension was diluted 100-fold, and 200 µl, 1.0 ml, and 3.0 ml of the diluted suspension was dropped on to nitrocellulose filters, immobilized by aspiration, and placed in sensor units. The response of the microorganism sensors storing the prepared clubroot fungus was confirmed using yeast extract as a substrate. The results are shown in Table 12 and Figure 2. A high positive correlation was found between the number of stored spores and the response of the microorganism sensor to yeast extract.

**[Table 12] Response of microorganism sensors storing clubroot fungus to yeast extract**

| Immobilized microorganism solution | Number of spores | Volume of added yeast extract | Δ n A |
|---|---|---|---|
| 200 µl | 5.6 x 10⁶ | 1.0 ml | 52 |
| 1.0 ml | 2.8 x 10⁷ | 1.0 ml | 162 |
| 3.0 ml | 8.4 x 10⁷ | 1.0 ml | 309 |

### [Example 7]

### Response of microorganism sensors to healthy and diseased soils.

Soils from Mr. Y's greenhouse and Mr. I's greenhouse in Nishine town, Iwate Prefecture, were used as healthy soil samples. A soil developing clubroot disease and a soil developing root rot disease from the Nagano Chushin Agricultural Experimental Station, and a soil developing root rot disease from Kawakami village, Nagano prefecture, were used as diseased soil samples.

The phytopathogenic microorganisms stored in the microorganism sensors were *Brassicaceae* clubroot fungus (*Plasmodiophora brassicae*) and the SN3B strain of lettuce root rot fungus (*Fusarium oxysporum* f. sp. *Lactucum*). The antagonistic microorganism stored in the microorganism sensors was the K12N strain of *Bacillus cereus.*

The amount of phytopathogenic microorganisms stored in each sensor was about 10-100 times of the density in common diseased soil. The amount of antagonistic microorganism was about 10-100 times of the density in common soil.

The results are shown in Tables 13 and 14.

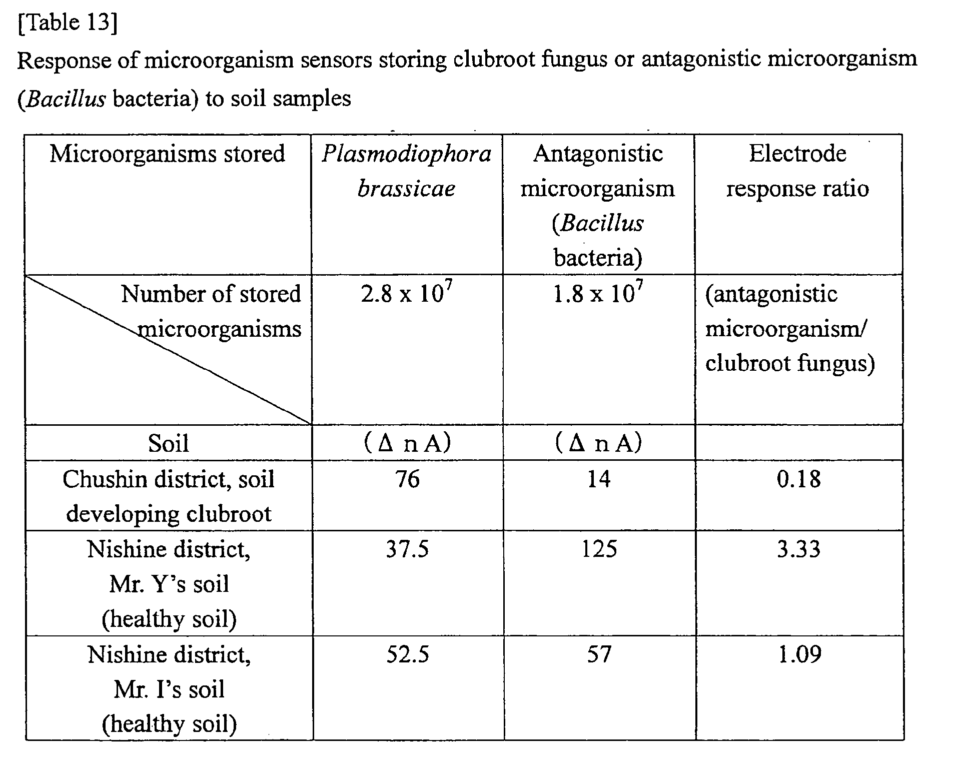

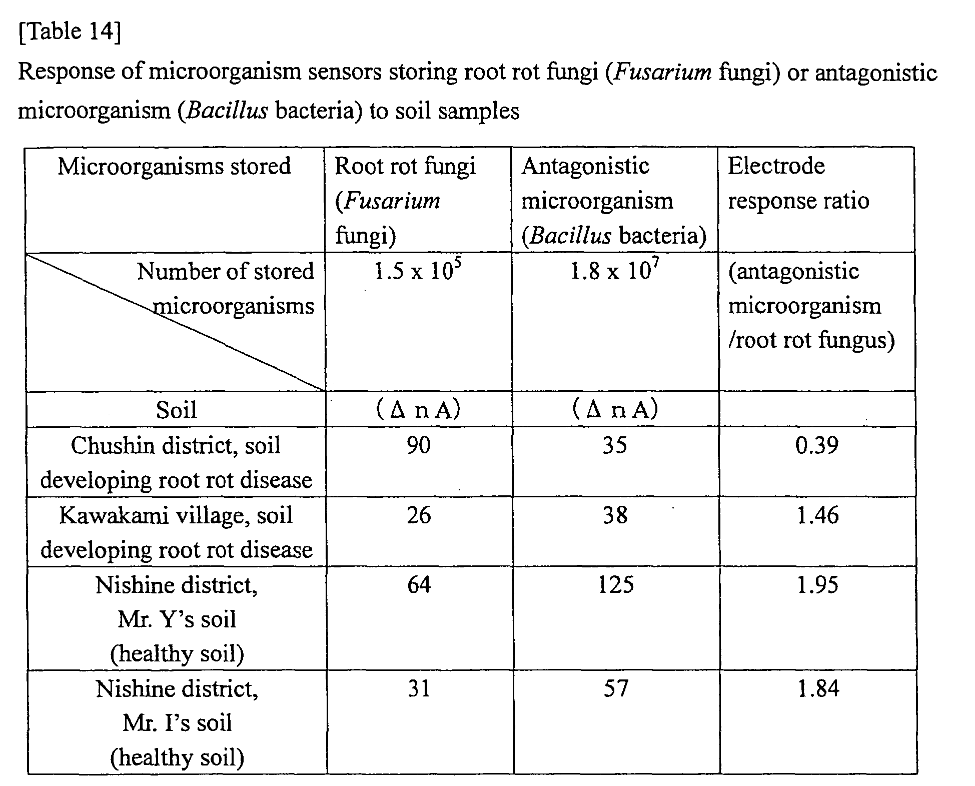

The results in Table 13 show that the electrode response ratio between the antagonistic microorganism and the clubroot fungus was low (0.18) in diseased soil, and high (3.33 and 1.09) in healthy soil.

The results in Table 14 show that the electrode response ratio between the antagonistic microorganism and the root rot microorganism was low (0.39 and 1.46) in diseased soil, and high (1.95 and 1.84) in healthy soil.

These results indicate that the electrode response ratio of antagonistic microorganism/phytopathogenic microorganism tends to be low in diseased soils, and high in healthy soils.

### [Example 8] Effect of soil sterilization and microorganism sensor response.

The Chushin Agricultural Experimental Station and surrounding district frequently suffer from *Brassicaceae* clubroot disease. Soil disinfection using chloropicrin and the like has provided only a temporary effect, and the disease has been found to reoccur soon after. This is generally characteristic of a soil damaged by continuous cropping.

However, when a soil artificially contaminated with lettuce root rot disease was prepared at the Chushin Agricultural Experimental Station, it was found that the first crop after inoculation developed the disease, but the second crop did not. Thus, when creating this root rot diseased soil, artificial inoculation of the pathogenic fungus was required every year. This is characteristic of a disease suppressive soil.

Lettuce root rot fungus has recently been found in the Kawakami village area, some areas of which have been cultivating lettuce for 30 years or more, and the disease is reportedly spreading every year. Although measures such as soil sterilization have been taken, no effective means for suppression has been found.

The above-described soil, which develops disease but recovers easily after chemical treatment and such (the soil artificially infected with root rot in the Chushin district = suppressive soil), and the soils in which disease tends to reoccur even after chemical treatment (the soil naturally infected with clubroot in the Chushin district, and the soil naturally infected with root rot in Kawakami village = soils damaged by continuous cropping) were examined to determine the effect of soil disinfection on microorganism sensor response.

**[Table 15] Effect of soil sterilization on the response of microorganism sensors storing microorganisms**

| Microorganisms stored | Clubroot fungus | Root rot fungus *(Fusarium)* | Antagonistic bacteria *(Bacillus)* | Electrode response ratio |
|---|---|---|---|---|
| | (ΔnA) | (ΔnA) | (ΔnA) | (Antagonistic microorganism/ pathogenic microorganism) |
| (Chushin district, soil diseased with clubroot) (soil damaged by continuous cropping) | | | | |
| Untreated area | 76 | | 14 | 0.18 |
| Sterilized area | 93 | | 30 | 0.32 |

| (Chushin district, soil diseased with root rot) (suppressive soil) | | | | |
|---|---|---|---|---|
| Untreated area | | 90 | 35 | 0.39 |
| Sterilized area | | 15 | 76 | 5.07 |

| (Kawakami village, soil diseased with root rot) (soil damaged by continuous cropping) | | | | |
|---|---|---|---|---|
| Untreated area | | 26 | 38 | 1.46 |
| Sterilized area | | 54 | 35.5 | 0.66 |

According to the results in Table 15, sterilization increased to 5.07 the electrode response ratio of antagonistic microorganism/pathogenic microorganism of the Chushin district soil, which was diseased with root rot but tended to recover easily upon chemical treatment and such, even after development of the disease by artificial inoculation. This indicates that in the sterilized soil the respiratory activity of the antagonistic microorganism was relatively higher than that of the pathogenic microorganism. Thus, this soil is an environment in which the antagonistic microorganism can grow more readily than the pathogenic microorganism (root rot fungus) when both microorganisms enter the soil after sterilization, and therefore the risk of disease recurrence is predicted to be low. The result supports the characteristics of a suppressive soil, where a disease induced by artificial inoculation of a pathogenic microorganism has disappeared by the second crop.

On the other hand, in the Chushin district soil naturally diseased with clubroot, and the Kawakami village soil naturally diseased with root rot, for which measures such as soil disinfection after disease occurrence tend to be temporary, the electrode response ratios of antagonistic microorganism/pathogenic microorganism in the sterilized soil were low, 0.32 and 0.66, respectively. These results indicated that the respiratory activity of the pathogenic microorganisms was higher than that of the antagonistic microorganism. Thus, these soils are an environment in which the pathogenic microorganism can grow more readily than the antagonistic microorganism when both microorganisms enter the soil after sterilization, and therefore the risk of disease recurrence is predicted to be high. This result supports results showing that even when soil disinfection is performed at the sites, it is ineffective and the disease readily reoccurs.

Based on these results, it is predicted that the higher the electrode response ratio of antagonistic microorganisms/pathogenic microorganisms, the lower the risk of disease occurrence; whereas the lower the electrode response ratio, the higher the risk of disease occurrence.

### [Example 9] Isolation of general soil microorganisms, their immobilization in a microorganism sensor, and confirmation of the sensor's response.

A soil sample from Mr. I's greenhouse in Nishine town, Iwate prefecture, was used as the healthy soil. A soil sample diseased with clubroot disease from the Nagano Chushin Agricultural Experimental Station, and a soil sample diseased with root rot from Kawakami village, Nagano prefecture were used as diseased soils. Soil microorganisms were isolated from each soil sample using the dilution plate method. Albumin agarose media and Rose Bengal agarose media were used for bacteria and fungi, respectively. Colonies appearing on the media were classified by color, shape outline, surface appearance, and size. Bacteria were classified into four colors, two outline shapes, four surface appearances, and three sizes, and into 96 patterns in total. Fungi were classified into seven colors, two outline shapes, four surface appearances, and three sizes, and into 168 patterns in total. The results are shown in Table 16.

**[Table 16] Characteristics of soil microorganisms isolated from various soil samples**

| | | Nishine district, Mr. I's soil | | Chushin district, soil diseased with clubroot | | Kawakami village, soil diseased with root rot | |
|---|---|---|---|---|---|---|---|
| | | Bacteria | Fungi | Bacteria | Fungi | Bacteria | Fungi |
| Number of Microorganisms (cfu/g of soil) | | 2.1x10⁷ | 9.7 x 10⁵ | 1.1 x 10⁷ | 4.7 x 10⁴ | 6.8 x 10⁷ | 3.9 x 10⁴ |
| Number of examined colonies | | 423 | 193 | 214 | 94 | 135 | 78 |

| Type | | 22 | 42 | 28 | 38 | 21 | 19 |
|---|---|---|---|---|---|---|---|
| Dominant species ratio(%) | 1 | 45.6 (00000) | 30.3* (00001) | 35.7 (00000) | 14.1* (21100) | 37.8 (10000) | 32.1* (21111) |
| Dominant species ratio(%) | 2 | 18.9* (11000) | 8.5 (30101) | 15.6* (10010) | 10.2 (21101) | 16.6* (11011) | 20.6 (21112) |
| Dominant species ratio(%) | 3 | 6.4 (01001) | 6.3 (00101) | 10.3 (10000) | 6.6 (32102) | 14.6 (11010) | 16.0 (21110) |
| Dominant species ratio(%) | 4 | 5.9 (11001) | 6.2 (30112) | 9.4 (10011) | 6.6 (01101) | 3.0 (10011) | 4.4 (01101) |
| Dominant species ratio(%) | other | 23.2 | 48.7 | 29.0 | 62.5 | 28.0 | 26.9 |

| | | | | | | | |
|---|---|---|---|---|---|---|---|
| *: Isolated, cultured, and used for a microorganism sensor. | | | | | | | |
| The numbers in parentheses indicate values for pattern classification (= strain number). | | | | | | | |

After classifying by pattern classification, a dominant species (single species) in each soil was isolated, cultured, and used to prepare microorganism sensors. The isolated microorganisms were not identified.

Next, each soil microorganism was placed in a microorganism sensor unit and their responses to yeast extract were examined. The results are shown in Table 17.

**[Table 17] Response to yeast extract of microorganism sensors storing (dominant type) soil microorganisms isolated from various soil samples**

| Soil for isolation | | Strain No. | OD660 | Amount of immobilized microorganism (µl) | Δ nA |
|---|---|---|---|---|---|
| Nishine district, Mr. I's soil (Healthy soil) | Bacterium | 11000 | 1.22 | 410 | 140 |
| | Fungus | 00001 | <0.01 | 700 | 21 |
| Chushin district (Soil diseased with clubroot) | Bacterium | 10010 | 0.71 | 700 | 147 |
| | Fungus | 21100 | 0.10 | 1,000 | 125 |
| Kawakami village (Soil diseased with root rot) | Bacterium | 11011 | 0.98 | 510 | 267 |
| | Fungus | 21111 | <0.01 | 1,000 | 136 |

The results in Table 17 show that all of the oxygen electrodes on which soil microorganisms were immobilized responded well to yeast extract.

### [Example 10] Comparison of the responses to diseased and healthy soils of microorganism sensors storing pathogenic microorganisms and those storing general soil microorganisms .

Clubroot fungus and root rot fungus were used as pathogenic microorganisms. Microorganisms isolated in Example 9 (the dominant species from each soil sample, six strains) were used as general soil microorganisms. Each microorganism was cultured, then placed in a microorganism sensor unit. Since some cultured microorganisms were not obtained in a sufficient amount, the responses were expressed as values relative to the response to yeast extract which was set as 100. The results are shown in Tables 18 and 19.

**[Table 18] Response of microorganism sensors to clubroot diseased soil and healthy soil**

| | Electrode response of immobilized microorganisms | | | Electrode response ratio | |
|---|---|---|---|---|---|
| | Club root fungus | General bacteria | General fungus | General bacteria/ Pathogenic fungus | General fungus/ Pathogenic fungus |
| Chushin district, Clubroot diseased soil | 51.9 nA | 1.5 nA | 39.1 nA | 0.029 | 0.753 |
| | (Strain No.) | (10010) | (21100) | | |
| (Number of microorganisms in soil) | 10^{6~7} | 1.7∗10⁶ | 6.6∗10³ | | |
| Nishine district, Mr. I's soil, (Healthy soil) | 58.0 nA | 38.2 nA | 54.8 nA | 0.659 | 0.945 |
| | (Strain No.) | (11000) | (00001) | | |
| (Number of microorganisms in soil) | ND* | 9.6∗10⁶ | 2.9∗10⁵ | | |

| | | | | | |
|---|---|---|---|---|---|
| ND*: not detected | | | | | |

According to the results in Table 18, in the Chushin district clubroot-diseased soil (disease severity: 100%), the density of the pathogenic microorganism (the clubroot fungus) was high (10⁶⁻⁷ cfu/g of soil), and further, the electrode response ratio of the microorganism sensor storing general bacterium or general fungus relative to the microorganism sensor storing pathogenic microorganism was low (0.029, 0.753). These results mean that the respiratory activity of the pathogenic microorganism was relatively high and that of the general soil microorganisms was relatively low. It was therefore predicted that the soil inclined toward disease spread. In fact, the occurrence of clubroot in the contaminated fields of the Chushin district continues to be severe.

On the other hand, in the healthy soil ofNishine district, the pathogenic microorganism (the clubroot fungus) was not detected, and the electrode response ratio was higher (0.659, 0.945) than for the clubroot-diseased soil from the Chushin district. Thus, the Nishine district soil was predicted to be resistant to the disease, and if the clubroot fungus did enter the soil, spread of the disease was predicted to be slower than for the clubroot-diseased soil in the Chushin district. In fact, clubroot has not been found in the Nishine field soil so far.

**[Table 19] Response of microorganism sensors to Fusarium wilt diseased soil and healthy soil**

| | Electrode response of immobilized microorganisms | | | Electrode response ratio | |
|---|---|---|---|---|---|
| | Root rot fungus | General bacterium | General fungus | General bacterium/ pathogenic microorganism | General fungus/ pathogenic microorganism |
| Kawakami village root rot diseased soil | 54.1 nA | 3.2 nA | 26.5 nA | 0.059 | 0.490 |
| | (Strain No.) | (11011) | (21111) | | |
| (Number of microorganisms) | 10³⁻⁴ | 2.6∗10⁷ | 1.3∗10⁴ | | |
| Nishine district Mr. I's soil (healthy soil) | 21.6 nA | 38.2 nA | 54.8 nA | 1.769 | 2.537 |
| | (Strain No.) | (11000) | (00001) | | |
| (Number of microorganisms) | ND | 9.6∗10⁶ | 2.9∗10⁵ | | |

According to the results in Table 19, in the Kawakami village root rot-diseased soil (disease incidence: 40%), the density of the pathogenic microorganism (root rot fungus) was high (10³⁻⁴ cfu/g soil). In addition, the respiratory activity of the pathogenic microorganism was relatively high, whereas that of the general soil microorganisms was relatively low, and therefore the electrode response ratio of microorganism sensors storing a general bacterium or general fungus was low (0.059 or 0.490, respectively) relative to that storing a pathogenic microorganism. Thus, the soil is predicted to incline towards disease spread. In fact, the area developing root rot in the Kawakami village district is increasing every year.

In contrast, no pathogenic microorganism (lettuce root rot fungus) was detected in the healthy soil of the Nishine district, and the electrode response ratio was significantly higher (1.769, 2.537) than that in the Kawakami village root rot-diseased soil. Thus, it is predicted that the Nishine district soil is highly resistant to the disease, and if root rot fungus did enter the soil, the risk of disease occurrence is predicted to be lower than for the Kawakami village root rot-diseased soil. In fact, root rot has not been found in the field site soil so far.

The electrode response ratio results for Examples 7, 8, and 10 (Tables 13, 14, 15, 18, and 19) are summarized in Tables 20 and 21.

**[Table 20] Electrode response ratios between a microorganism sensor storing clubroot fungus and sensors storing a general microorganism, root rot incidence of each soil, and density of the pathogenic microorganism**

| | | | Electrode response ratio | | | Sterilized soil |
|---|---|---|---|---|---|---|
| | Disease severity (%) | Pathogenic microorganism density (spores/g soil) | vs. antagonistic microorganism | vs. general bacterium | vs. general fungus | vs. antagonistic microorganism |
| Chushin district, clubroot diseased soil | 100 | 10^{6~7} | 0.18 (K12N) | 0.029 (10010) | 0.753 (21100) | 0.32 (K12N) |
| Nishine district, Mr. I's healthy soil | 0 | ND | 1.09 (K12N) | 0.659 (11000) | 0.945 (00001) | |
| Nishine district, Mr. Y's healthy soil | 0 | ND | 3.33 (K12N) | | | |

| | | | | | | |
|---|---|---|---|---|---|---|
| ND: not detected; strain numbers appear in parentheses. | | | | | | |

**[Table 21] Electrode response ratios between a microorganism sensor storing root rot fungus and sensors storing a general microorganism, root rot incidence of each soil, and density of the pathogenic microorganism**

| | | | Electrode response ratio | | | Sterilized soil |
|---|---|---|---|---|---|---|
| | Disease severity (%) | Pathogenic microorganism density (spores/g soil) | vs. antagonistic microorganism | vs. general bacterium | vs. general fungus | vs. antagonistic microorganism |
| Chushin district, root rot diseased soil (Suppressive soil) | 30 | 10³ | 0.39 (K12N) | | | 5.07 (K12N) |
| Kawakami village, root rot diseased soil | 40 | 10^{3~4} | 1.46 (K12N) | 0.059 (11011) | 0.490 (21111) | 0.66 (K12N) |
| Nishine district, Mr. I's healthy soil | 0 | ND | 1.84 (K12N) | 1.769 (11000) | 2.537 (00001) | |
| Nishine district, Mr. Y's healthy soil | 0 | ND | 1.95 (K12N) | | | |

| | | | | | | |
|---|---|---|---|---|---|---|
| ND: not detected; strain numbers appear in parentheses | | | | | | |

Based on these results, it became possible to predict whether the risk of disease occurrence in a soil environment is low or high by using an antagonistic soil microorganism or a dominant species (general soil bacterium and general soil fungus) isolated from a field site soil to examine the electrode response ratio relative to a pathogenic microorganism (general soil microorganism/pathogenic microorganism). These predicted results were well consistent with the results of research on disease occurrence in the field sites.

### [Example 11] Control of tomato bacterial wilt by Pythium fungi and use of a microorganism sensor to predict the same.

On May 29, 2003, *Pythium oligandrum* strain MMR2 and various materials were added to nursery soil (Modular Seed, William Sinclair Holdings plc.), this was added to a 128-cell tray, and tomato seeds (cultivar: "Reika") were seeded. On June 11, the seedlings were repotted from the cell tray into 5-cm connected pots (cultivation soil: Supermix-A, distributor: Sakata Seed Co.), and tomato bacterial wilt bacteria *(Ralstonia solanacearum)* was artificially inoculated. After one month of cultivation, the disease severities and the percentage of diseased plants were examined (Table 22).

**[Table 22] Test of bacterial wilt control Cultivar: "Reika" Date of examination: July 11, 2003**

| Treatment | Material | Number of plants | Disease index | | | | | Disease severity | Percentage of diseased plants | Protective value |
|---|---|---|---|---|---|---|---|---|---|---|
| Microorganism (strain No.) | No. | | 0 | 1 | 2 | 3 | 4 | | | |
| Untreated | 1 | 20 | 1 | 5 | 4 | 5 | 5 | 60.0 | 70.0 | |
| *Pythium* fungus (MMR2) | 5 | 20 | 0 | 10 | 5 | 3 | 2 | 46.3 | 50.0 | 22.9 |

*Pythium oligandrum* strain MMR2 is an antagonistic microorganism which is parasitic to fungi and capable of inducing disease resistance. Administration of the microorganism reduced the disease severity and the percentage of diseased plants to 46.3 and 50.0, respectively. The protective value was 22.9, indicating a controlling effect on *Ralstonia solanacearum.*

The number of infested microorganisms was 7 x 10⁷ cfu/g soil for *Ralstonia solanacearum,* and 1 x 10⁴ cfu/g soil for the *Pythium* sp.

*Ralstonia solanacearum* was cultured at 30°C for one to two days on dishes containing TTC agarose media. The *Pythium* fungus was cultured with shaking in Erlenmeyer flasks containing V8 juice media at 25°C for one month. Both microorganisms were collected and placed in separate microorganism sensor units.

Both of the microorganism-immobilized electrodes were confirmed to respond to yeast extract *(Ralstonia solanacearum:* 196 nA; *Pythium oligandrum:* 23 nA).

The response of both microorganism sensors storing the microorganisms to the cultivation soil was examined. The result is shown in Table 23.

**[Table 23] Response of Ralstonia solanacearum and Pythium fungus**

| | | *Ralstonia solanacearum* | *Pythium* fungus | Electrode response ratio |
|---|---|---|---|---|
| | | (ΔnA) | (ΔnA) | *Pythium* fungus/ *R. solanacearum* |
| Nursery soil | Modular Seed | 38 | 77 | 2.03 |
| Potting soil | Supermix-A | 10 | 67 | 6.70 |

Based on the results shown in Table 23, it is predicted that when inoculated to nursery soil and potting soil the *Pythium* fungus will adapt to the soil environment and grow better than *Ralstonia solanacearum.* This is considered to result in a reduced occurrence of tomato bacterial wilt.

Thus, the results of predictions using the microorganism sensors of the present invention were found to be well consistent with the results of actual control tests.

### [Example 12] Influence of the use of various microorganism materials on soil diseases and prediction of disease mitigation using a microorganism sensor.

On May 29 and August 26, 2003, various microorganism materials were added to cultivation soils (Metromix, Scotts-Sierra Horticultural Products Company; or Modular Seed), and then added to 128-cell trays. Tomato (cultivar: Reika), lettuce (cultivar: Red wave and Salinas), komatsuna (cultivar: Kiyosumi), and spinach (cultivar: Platon) were seeded. On June 11 and September 9, the tomato, lettuce, and spinach were repotted from the cell trays into 20-well or 24-well connected pots. "Supermix-A" was used as the potting soil. The komatsuna were potted in 40-well connected pots. A mixture of clubroot-diseased soil from the Nagano Chushin Agricultural Experimental Station, Supermix-A, and soil for raising rice seedlings was used at 2:1:1 as the potting soil.

Phytopathogenic microorganisms were inoculated, and disease occurrence was observed over time.

The inoculated density of microorganism materials and phytopathogenic microorganisms was calculated from the microorganism concentration prior to inoculation to the soil.

Of the microorganism materials, Bio-21 (Sakata Seed Co.) is a *Bacillus* sp., and MMR3 is a *Pythium* sp. parasitic to fungi and capable of inducing disease resistance, which was provided from National Agricultural Research Center for Hokkaido Region.

Research on the occurrence of tomato bacterial wilt was conducted on July 4 and September 18. The disease severity was determined using the following five criteria: 0: no disease; 1: parts of leaves are wilted or yellowed (slight occurrence); 2: about 50% of the leaves or less are wilted (moderate occurrence); 3: 50% of the leaves or more are wilted and part of the leaves blighted (severe occurrence); 4: blighted. The disease severity, percentage of diseased plants, and protective values were then calculated. The results are shown in Table 24.

The severity of lettuce root rot occurrence was examined on July 11 and October 6, by cutting the vessels. The disease severity was determined using the following five criteria: 0: no browning of vessel; 1: slightly browned vessel (slight occurrence); 2: browned vessel (moderate occurrence); 3: markedly browned vessel and leaves in the aerial part are yellowed or wilted (severe occurrence); 4: blighted. The disease severity, percentage of diseased plants, and protective values were then calculated. The results are shown in Table 25.

The severity of spinach wilt occurrence was examined on October 6, by cutting the vessels. The disease severity was determined using the same criteria as for lettuce, and the results are shown in Table 26.

The severity of komatsuna clubroot occurrence was determined on July 11 and October 6, using the degree of root knot formation in washed roots. The disease severity was determined using the following five criteria: 0: no root knot formation; 1: slight root knot formation in capillary roots (light occurrence); 2: formation of small knots in lateral or main roots (moderate occurrence); 3: formation of large root knots (severe occurrence); 4: blighted. The disease severity, percentage of diseased plants, and protective values were then calculated. The results are shown in Table 27.

**[Table 24] The effect of various microorganism materials in controlling tomato bacterial wilt (1) Seeded on May 29, examined on July 4 (cultivar: Reika)**

| Microorganism material | Microorganism density | Disease index | | | | | | | |
|---|---|---|---|---|---|---|---|---|---|
| | cfu/g soil | 0 | 1 | 2 | 3 | 4 | Disease severity | Percentage of diseased plants | Protective value |
| Untreated | 7 x 10⁷ | 11 | 2 | 3 | 1 | 3 | 28.75 | 35 | |
| Bio-21 | 5 x 10⁷ | 14 | 1 | 3 | 1 | 1 | 17.50 | 25 | 39.1 |
| Cerafarm | 5 x 10⁷ | 12 | 4 | 41 | 3 | 0 | 18.75 | 20 | 34.8 |
| Hardin-L | ND | | | | | | | | |
| Mycostop | 1 x 10⁴ | 16 | 2 | 1 | 1 | 0 | 8.75 | 10 | 69.6 |
| HAI00377 | 2 x 10⁵ | 14 | 1 | 3 | 2 | 0 | 16.25 | 25 | 43.5 |
| MMR3 | 1 x 10⁴ | 18 | 1 | 1 | 0 | 0 | 3.75 | 5 | 87.0 |
| (2) Seeded on August 26, examined on September 18 (cultivar: "Reika") | | | | | | | | | |

| Microorganism material | Microorganism density*⁾ | Disease index | | | | | | | |
|---|---|---|---|---|---|---|---|---|---|
| | cfu/g soil | 0 | 1 | 2 | 3 | 4 | Disease severity | Percentage of diseased plants | Protective value |
| Untreated | 7 x 10⁷ | 0 | 0 | 0 | 1 | 23 | 99.0 | 100.0 | |
| Bio-21 | 5 x 10⁷ | 2 | 0 | 1 | 10 | 11 | 79.2 | 91.7 | 20.0 |
| Cerafarm | 5 x 10⁷ | 0 | 0 | 0 | 8 | 16 | 91.7 | 100.0 | 7.4 |
| Hardin-L | 5 x 10⁵ | 0 | 0 | 0 | 0 | 24 | 100.0 | 100.0 | -1.1 |
| Mycostop | 1 x 10⁴ | 1 | 2 | 5 | 5 | 11 | 74.0 | 87.5 | 25.3 |
| HAI00377 | 2 x 10⁵ | 0 | 0 | 0 | 0 | 24 | 100.0 | 100.0 | -1.1 |
| MMR3 | 1 x 10⁴ | 2 | 0 | 1 | 5 | 16 | 84.4 | 91.7 | 14.7 |

| | | | | | | | | | |
|---|---|---|---|---|---|---|---|---|---|
| *⁾: Microorganism density column: "Untreated" shows the density of the phytopathogenic microorganism; the others show the microorganism density of each microorganism material (antagonistic microorganism). | | | | | | | | | |

**[Table 25] Effect of microorganism materials in controlling lettuce root rot (1) Seeded on May 29, examined on July 11 (cultivar: Red-wave)**

| Microorganism material | Microorganism density*⁾ | Disease index | | | | | | | |
|---|---|---|---|---|---|---|---|---|---|
| | cfu/g soil | 0 | 1 | 2 | 3 | 4 | Disease severity | Percentage of diseased plants | Protective value |
| Untreated | 7 x 10² | 0 | 12 | 20 | 7 | 33 | 71.2 | 83.3 | |
| Bio-21 | 5 x 10⁷ | 0 | 6 | 6 | 1 | 11 | 67.7 | 75.0 | 4.9 |
| Cerafarm | 5 x 10⁷ | 0 | 4 | 6 | 3 | 11 | 71.9 | 83.3 | -1.0 |
| Hardin-L | 5 x 10⁵ | 0 | 4 | 8 | 2 | 10 | 68.8 | 83.3 | 3.4 |
| Mycostop | 1 x 10⁴ | 0 | 2 | 3 | 3 | 16 | 84.4 | 91.7 | -18.5 |
| HAI00377 | 2 x 10⁵ | 0 | 5 | 12 | 2 | 5 | 57.3 | 79.2 | 19.5 |
| MMR3 | 1 x 10⁴ | 0 | 4 | 11 | 3 | 6 | 61.5 | 83.3 | 13.7 |
| (2) Seeded on August 26, examined on October 6 (cultivar: Salinas) | | | | | | | | | |

| Microorganism material | Microorganism density*⁾ | Disease index | | | | | | | |
|---|---|---|---|---|---|---|---|---|---|
| | cfu/g soil | 0 | 1 | 2 | 3 | 4 | Disease severity | Percentage of diseased plants | Protective value |
| Untreated | 7 x 10² | 1 | 8 | 8 | 3 | 4 | 51.0 | 62.5 | |
| Bio-21 | 5 x 10⁷ | 1 | 15 | 6 | 2 | 0 | 34.4 | 33.3 | 32.7 |
| Cerafarm | 5 x 10⁷ | 0 | 11 | 10 | 3 | 0 | 41.7 | 54.2 | 18.4 |
| Hardin-L | 5 x 10⁵ | 0 | 16 | 6 | 1 | 1 | 36.5 | 33.3 | 28.6 |
| Mycostop | 1 x 10⁴ | 2 | 6 | 13 | 2 | 1 | 43.8 | 66.7 | 14.3 |
| HAI00377 | 2 x 10⁵ | 2 | 11 | 6 | 4 | 1 | 40.6 | 45.8 | 20.4 |
| MMR3 | 1 x 10⁴ | 2 | 13 | 8 | 1 | 0 | 33.3 | 37.5 | 34.7 |

| | | | | | | | | | |
|---|---|---|---|---|---|---|---|---|---|
| *⁾ : Microorganism density column: "Untreated" shows the density of the phytopathogenic microorganism; the others show the microorganism density of each microorganism material (antagonistic microorganism). | | | | | | | | | |

**[Table 26] Effect of various microorganism materials in controlling spinach wilt Seeded on August 26, examined on October 6 (cultivar: Platon)**

| Microorganism material | Microorganism density*⁾ | Disease index | | | | | | | |
|---|---|---|---|---|---|---|---|---|---|
| | cfu/g soil | 0 | 1 | 2 | 3 | 4 | Disease severity | Percentage of diseased plants | Protective value |
| Untreated | 3 x 10³ | 0 | 18 | 3 | 1 | 2 | 36.5 | 25.0 | |
| Bio-21 | 5 x 10⁷ | 2 | 19 | 1 | 0 | 2 | 30.2 | 12.5 | 17.1 |
| Cerafarm | 5 x 10⁷ | 0 | 11 | 11 | 2 | 0 | 40.6 | 54.2 | -11.4 |
| Hardin-L | 5 x 10⁵ | 2 | 10 | 5 | 3 | 4 | 46.9 | 50.0 | -28.6 |
| Mycostop | 1 x 10⁴ | 3 | 15 | 3 | 0 | 3 | 34.4 | 25.0 | 5.7 |
| HAI00377 | 2 x 10⁵ | 3 | 16 | 2 | 2 | 1 | 31.3 | 20.8 | 14.3 |
| MMR3 | 1 x 10⁴ | 2 | 17 | 3 | 0 | 1 | 28.1 | 17.4 | 22.9 |

| | | | | | | | | | |
|---|---|---|---|---|---|---|---|---|---|
| *⁾: Microorganism density column: "Untreated" shows the density of the phytopathogenic microorganism; the others show the microorganism density of each microorganism material (antagonistic microorganism). | | | | | | | | | |

**[Table 27] Effect of various microorganism materials in controlling komatsuna clubroot (1) Seeded on May 29, examined on July 11 (cultivar: Kiyosumi)**

| Microorganism material | Microorganism density*⁾ | Disease index | | | | | | | |
|---|---|---|---|---|---|---|---|---|---|
| | cfu/g soil | 0 | 1 | 2 | 3 | 4 | Disease severity | Percentage of diseased plants | Protective value |
| Untreated | 3 x 10⁶ | 1 | 6 | 9 | 3 | 0 | 41.3 | 90.0 | |
| Bio-21 | 5 x 10⁷ | 6 | 7 | 2 | 5 | 0 | 32.5 | 70.0 | 21.2 |
| Cerafarm | 5 x 10⁷ | 7 | 6 | 4 | 3 | 0 | 28.8 | 65.0 | 30.3 |
| Hardin-L | 5 x 10⁵ | 10 | 5 | 5 | 0 | 0 | 18.8 | 50.0 | 54.5 |
| Mycostop | 1 x 10⁴ | 4 | 6 | 1 | 9 | 0 | 43.8 | 80.0 | -6.1 |
| HAI00377 | 2 x 10⁵ | 5 | 6 | 7 | 2 | 0 | 32.5 | 75.0 | 21.2 |
| MMR3 | 1 x 10⁴ | 3 | 7 | 6 | 4 | 0 | 38.8 | 85.0 | 6.1 |
| (2) Seeded on August 26, examined on October 6 (cultivar: Kiyosumi) | | | | | | | | | |

| Microorganism material | Microorganism density*⁾ | Disease index | | | | | | | |
|---|---|---|---|---|---|---|---|---|---|
| | cfu/g soil | 0 | 1 | 2 | 3 | 4 | Disease severity | Percentage of diseased plants | Protective value |
| Untreated | 3 x 10⁶ | 0 | 0 | 13 | 19 | 8 | 71.9 | 100.0 | |
| Bio-21 | 5 x 10⁷ | 1 | 4 | 16 | 19 | 0 | 58.1 | 87.5 | 19.1 |
| Cerafarm | 5 x 10⁷ | 0 | 1 | 9 | 28 | 2 | 69.4 | 97.5 | 3.5 |
| Hardin-L | 5 x 10⁵ | 0 | 3 | 7 | 29 | 1 | 67.5 | 92.5 | 6.1 |
| Mycostop | 1 x 10⁴ | 1 | 1 | 8 | 27 | 3 | 68.8 | 95.0 | 4.3 |
| HAI00377 | 2 x 10⁵ | 0 | 0 | 8 | 29 | 3 | 71.9 | 100.0 | 7.8 |
| MMR3 | 1 x 10⁴ | 0 | 2 | 11 | 23 | 4 | 68.1 | 95.0 | 5.2 |

| | | | | | | | | | |
|---|---|---|---|---|---|---|---|---|---|
| *⁾ : Microorganism density column: "Untreated" shows the density of the phytopathogenic microorganism; the others show the microorganism density of each microorganism material (antagonistic microorganism). | | | | | | | | | |

To prepare microorganism sensors storing various microorganism materials and phytopathogenic microorganisms, bacteria were cultured at 30°C for 12 days in L broth media with shaking, and fungi were cultured at 25°C for seven days in PD broth media with shaking.

The bacteria and fungi were collected, washed, immobilized on nitrocellulose filters, and placed in sensor units.

The response of each microorganism sensor to potting soil and the electrode response ratio of antagonistic microorganism/pathogenic microorganism were determined, and the results are shown in Table 28.

**[Table 28] Electrode response of microorganism sensors storing various microorganism materials and phytopathogenic microorganisms**

| | Electrode response (ΔnA) | | Electrode response ratio (antagonistic microorganism/pathogenic microorganism) | | | |
|---|---|---|---|---|---|---|
| | Nursery soil (Supermix-A) | Clubroot diseased soil | *Ralstonia solanacearum* | *Fusarium oxysporum* f. sp. *Lactucum* | *Plasmodiophora brassicae* | *Fusarium oxysporum* f. sp. *spinaciae* |
| Bio-21 | 18.0 | 29.0 | 1.80 | 0.57 | 1.76 | 0.87 |
| Cerafarm | 8.0 | 42.0 | 0.80 | 0.25 | 2.55 | 0.39 |
| Hardin-L | 16.5 | 16.5 | 1.65 | 0.52 | 1.00 | 0.80 |
| Mycostop | 4.5 | 18.0 | 0.45 | 0.14 | 1.09 | 0.22 |
| HAI00377 | 29.0 | 30.0 | 2.90 | 0.92 | 1.82 | 1.40 |
| MMR3 | 23.5 | 14.5 | 2.35 | 0.74 | 0.88 | 1.14 |
| *R solanacearum* | 10.0 | - | | | | |
| *F. oxysporum f.* sp. *Lactucum* | 31.7 | - | | | | |
| *P. brassicae* | - | 16.5 | | | | |
| *F. oxysporum* f. sp. *spinaciae* | 20.7 | - | | | | |

The introduced amount of microorganism materials was determined in principle according to the methods recommended by the respective manufacturers or developers. Thus in some cases the number of antagonistic microorganisms introduced differed greatly from the inoculated number of pathogenic microorganisms.

A difference in microorganism density of about ten times is not often problematic for soil microorganisms, but a difference of 100 times or more needs to be taken into consideration.

Thus, the disease mitigating effect was determined according to the electrode response ratio corrected by the microbial number differences between both populations as shown in the following table.

**[Table 29] Standard for correction by microbial number differences when predicting the disease mitigating effect of introduced microorganism materials**

| Correction by microbial number differences | Electrode response ratio | | | |
|---|---|---|---|---|
| | 0.4< | 0.4~0.6 | 0.6~2.0 | 2.0< |
| Pathogenic microorganism>> antagonistic microorganism | Ineffective | | | Effective |
| Pathogenic microorganism ≒ antagonistic microorganism | Ineffective | | Effective | |
| Pathogenic microorganism<< antagonistic microorganism | Ineffective | Effective | | |

| | | | | |
|---|---|---|---|---|
| >> or <<: indicates 100 times or more difference in the number of microorganisms. | | | | |

The effects of microorganism materials were predicted based on Table 29, and the predictions were compared with the results of actual controlling effects.

Protective values of 10.0 or more were considered to have a controlling effect. The results are shown in Table 30.

**[Table 30] Comparison between the controlling effects of microorganism materials and the results of using biosensors to predict controlling effects**

| (1) Tomato bacterial wilt | | | | | |
|---|---|---|---|---|---|
| Microorganism material | Protective value (mean) | Electrode response ratio | Correction by microbial number differences | Biosensor prediction | Correlation |
| Bio21 | 29.6 | 1.80 | | Effective | ⊚ |
| Cerafarm | 21.1 | 0.8 | | Effective | ⊚ |
| Hardin-L | -1.1 | 1.65 | Pathogenic microorganism>> Antagonistic microorganism | Ineffective | ⊚ |
| Mycostop | 47.5 | 0.45 | Pathogenic microorganism>> Antagonistic microorganism | Ineffective | × |
| HAI00377 | 21.2 | 2.90 | Pathogenic microorganism>> Antagonistic microorganism | Effective | ⊚ |
| MMR3 | 50.9 | 2.35 | Pathogenic microorganism>> Antagonistic microorganism | Effective | ⊚ |

| (2) Lettuce root rot | | | | | |
|---|---|---|---|---|---|
| | Protective value (mean) | Electrode response ratio | Correction by microbial number differences | Biosensor prediction | Correlation |
| Bio21 | 18.8 | 0.57 | Pathogenic microorganism<< Antagonistic microorganism | Effective | ⊚ |
| Cerafarm | 8.7 | 0.25 | Pathogenic microorganism<< Antagonistic microorganism | Ineffective | ⊚ |
| Hardin-L | 16.0 | 0.52 | Pathogenic microorganism<< Antagonistic microorganism | Effective | ⊚ |
| Mycostop | -2.1 | 0.14 | | Ineffective | ⊚ |
| HAI00377 | 20.0 | 0.92 | Pathogenic microorganism<< Antagonistic microorganism | Effective | ⊚ |
| MMR3 | 24.2 | 0.74 | | Effective | ⊚ |

| (3) *Brassicaceae* clubroot | | | | | |
|---|---|---|---|---|---|
| | Protective value (mean) | Electrode response ratio | Correction by microbial number differences | Biosensor prediction | Correlation |
| Bio21 | 20.2 | 1.76 | | Effective | ⊚ |
| Cerafarm | 16.9 | 2.55 | | Effective | ⊚ |
| Hardin-L | 30.3 | 1.00 | | Effective | ⊚ |
| Mycostop | -0.9 | 1.09 | Pathogenic microorganism >> Antagonistic microorganism | Ineffective | ⊚ |
| HAI00377 | 14.5 | 1.82 | | Effective | ⊚ |
| MMR3 | 5.7 | 0.88 | Pathogenic microorganism>> Antagonistic microorganism | Ineffective | ⊚ |

| (4) Spinach wilt | | | | | |
|---|---|---|---|---|---|
| | Protective value | Electrode response ratio | Correction by microbial number differences | Biosensor prediction | Correlation |
| Bio21 | 17.1 | 0.87 | Pathogenic microorganism<< Antagonistic microorganism | Effective | ⊚ |
| Cerafarm | -11.4 | 0.39 | Pathogenic microorganism<< Antagonistic microorganism | Ineffective | ⊚ |
| Hardin-L | -28.6 | 0.80 | Pathogenic microorganism<< Antagonistic microorganism | Effective | × |
| Mycostop | 5.7 | 0.22 | | Ineffective | ⊚ |
| HAI00377 | 14.3 | 1.40 | | Effective | ⊚ |
| MMR3 | 22.9 | 1.14 | | Effective | ⊚ |

A protective value of 10 or higher was determined to have a controlling effect.

The electrode response ratio was the ratio of the electrode responses of both microorganism sensors storing antagonistic microorganisms and pathogenic microorganisms.

Correction by microbial number differences (<< or >>) was performed when the difference in the number of microorganisms was 100 times or more.

**[Table 31] Correlation between the predicted results and control test results.**

| | | |
|---|---|---|
| ⊚ | × | Total |
| 22 | 2 | 24 |

In addition, comparisons of the disease severity and the protective values in each of the two tests (1) and (2) in Tables 24, 25, and 27 revealed a clear tendency towards a higher disease severity resulting in reduced protective values (in all data except Mycostop in Table 27). In particular, the controlling effect may be masked under conditions of severe occurrence, in which the disease severity is 90 or higher. For example, the electrode response ratio of the HAI00377 strain relative to *Ralstonia solanacearum* was significantly high (2.90, Table 28), and the strain was determined to have a controlling effect. However, since the disease severity was 100.0 (99.0 for no treatment) in Table 24 (2), indicating conditions of severe occurrence, the resulting protective value was -1.1, and therefore the controlling effect was considered to be masked. In such cases, it is desirable to repeat the test under conditions where the disease severity is 90% or lower.

The correlation between the predictions using biosensors and the results of actual prevention tests indicates that 22 predictions from 24 tests were accurate. Thus, the accuracy of prediction was as high as 91.7%, indicating that the prediction method is highly reliable.

### Industrial Applicability

Use of the present invention's microorganism sensors for predicting the dynamics of soil microorganisms makes it possible to analyze soil microorganism dynamics, in particular, it enables the dynamics of soil-borne phytopathogenic microorganisms and general soil microorganisms (including antagonistic microorganisms) to be predicted in field soils. Further, it enables early prediction of the risk of disease occurrence in field soils, which in turn enables early control measures. The present invention can not only predict the occurrence of a disease in agricultural fields where the disease is already present, it can also predict the future risk of disease occurrence in agricultural fields where the disease has not yet occurred. In addition, proposals for soil improvements can be linked to the results of these predictions.

By suspending soil samples from various field sites directly in a solvent such as water, and applying this supernatant to the biosensors of this invention, anyone can quickly and simply determine whether a soil microorganism of interest can adapt to and grow in that soil environment, without requiring any special treatments or techniques.

## Claims

1. A method for measuring the growth potential of a soil microorganism in a soil, comprising the steps of:
(a) contacting a soil suspension of a soil to be measured with multiple sensors, each of which comprise a unit comprising an oxygen electrode, a housing section that stores a soil microorganism, and an immobilizing member, wherein the housing section of each sensor stores a different soil microorganism; and
(b) measuring the differences in a decrease or rate of decrease of output electric current for each of the sensors.

2. A method for evaluating the risk of the occurrence or spread of a soil disease caused by a soil-borne phytopathogenic microorganism, comprising the steps of:
(a) contacting a soil suspension of a soil to be measured with a sensor which comprises a unit comprising an oxygen electrode, a housing section that stores a general soil microorganism, and an immobilizing member, and with a sensor which comprises a unit comprising an oxygen electrode, a housing section that stores a soil-borne phytopathogenic microorganism, and an immobilizing member; and
(b) measuring a decrease or rate of decrease in output electric current for each of the sensors,
wherein the risk of occurrence or spread of the soil disease caused by the soil-borne phytopathogenic microorganism in the soil is determined to be low when the decrease or rate of decrease of output current in the case of the general soil microorganism is significantly higher than that in the case of the soil-borne phytopathogenic microorganism.

3. A method for evaluating an effect of a general soil microorganism in controlling a soil disease caused by a soil-borne phytopathogenic microorganism, comprising the steps of:
(a) contacting a soil suspension of a soil to be measured with a sensor which comprises a unit comprising an oxygen electrode, a housing section that stores a general soil microorganism, and an immobilizing member, and also contacting the soil suspension of the soil to be measured with a sensor which comprises a unit comprising an oxygen electrode, a housing section that stores a soil-borne phytopathogenic microorganism, and an immobilizing member; and
(b) measuring a decrease or rate of decrease in output electric current for each of the sensors,
wherein the general soil microorganism is determined to have an effect in controlling the soil disease caused by the soil-borne phytopathogenic microorganism in the soil when the decrease or rate of decrease of output current in the case of the general soil microorganism is significantly higher than that in the case of the soil-borne phytopathogenic microorganism.

4. A kit used for the method of claim 1, comprising multiple sensors each of which comprise a unit comprising an oxygen electrode, a housing section that stores a soil microorganism, and an immobilizing member, wherein the housing section of each sensor stores a different soil microorganism.

5. A kit used for the method of claim 2 or 3, comprising a sensor that comprises a unit comprising an oxygen electrode, a housing section that stores a general soil microorganism, and an immobilizing member; and comprising a sensor which comprises a unit comprising an oxygen electrode, a housing section that stores a soil-borne phytopathogenic microorganism, and an immobilizing member.
